# EUROPÄISCHE PATENTSCHRIFT

(11) **EP 0 476 670 B1**
(45) Veröffentlichungstag und Bekanntmachung des Hinweises auf die Patenterteilung: **12.05.1999**
(21) Anmeldenummer: 91115974.7
(22) Anmeldetag: 19.09.1991
(51) Int. Cl.: C12N 15/55, C12Q 1/34, C12N 1/21

(54) **N-Carbamoylsarcosin-Amidohydrolase, kloniert**
Cloned N-carbamoylsarcosine-amidohydrolase
N-carbamoylsarcosine-amidohydrolase clonée

(30) Priorität: 20.09.1990 DE 4029844
(43) Veröffentlichungstag der Anmeldung: 25.03.1992
(73) Patentinhaber: Roche Diagnostics GmbH, 68298 Mannheim (DE)
(72) Erfinder: Burtscher, Helmut, Dr., W-8121 Habach (DE); Schumacher, Günther, Dr., W-8139 Bernried (DE)
(74) Vertreter: Huber, Bernhard, Dipl.-Chem.

(56) Entgegenhaltungen:
- EP-A- 0 112 571
- EP-A- 0 154 269
- EP-A- 0 437 254
- CHEMICAL ABSTRACTS, vol. 109, no. 11, 12. September 1988, Columbus, Ohio, US; abstract no. 89041D, SIEDEL, J. ET AL.: 'Fully enzymatic colorimetric assay of serum and urine creatinine wich obviates the need for sample blank measurements' Seite 344; & Anal. Lett., 21(6), 1988, 1009-17
- NUCLEIC ACID RESEARCH Bd. 14, Nr. 16, Seite 6770 MCCLAEN 'Variants in clones of gene-maschine-synthesized oligodeoxynucleotides'

## Beschreibung

Das Enzym N-Carbamoylsarcosin-Amidohydrolase (CSHase) wird zur Bestimmung des Creatiningehaltes in Flüssigkeiten benötigt. Creatinin ist ein wesentlicher Parameter für die Nierendiagnostik. Weltweit werden ca. 1 Milliarde Tests pro Jahr durchgeführt. Die kostengünstige Bereitstellung des Enzyms CSHase sowie die Möglichkeit einer problemlosen Fermentation sind daher Grundvoraussetzungen für die Herstellung eines diagnostischen Kits zur Creatinin-Bestimmung.

CSHase ist ein Protein mit einem Molekulargewicht von ca. 35 kD (Untereinheit), einer K_{M} von 3 x 10⁻³ mol/l und einer spezifischen Aktivität von 2 U/mg. Bisher wurde die CSHase aus Arthrobacter gewonnen (EC 3.5.1.59). Nachteil dieses Verfahrens sind die komplexen Nährstoffansprüche von Arthrobacter und die bei der Anreicherung erhaltenen geringen Enzymaktivitäten von 100 bis 200 U/l.

Aufgabe der vorliegenden Erfindung war es daher, die Nachteile des Standes der Technik mindestens teilweise zu beseitigen, indem ein besseres Anreicherungsverfahren für CSHase bereitgestellt wird.

Gegenstand der vorliegenden Erfindung ist eine rekombinante DNA, welche die in SEQ ID NO. 1 dargestellte Sequenz enthält, wobei die DNA-Sequenz für ein Protein mit einer N-Carbamoylsarcosin-Amidohydrolase-Aktivität kodiert.

Die in SEQ ID NO: 1 dargestellte erfindungsgemäße DNA kodiert für ein Protein mit einer N-Carbamoylsarcosin-Amidohydrolase-Aktivität, das die in SEQ ID NO: 2 dargestellte Aminosäuresequenz aufweist. Daneben umfaßt die vorliegende Erfindung auch eine DNA-Sequenz, die der in SEQ ID NO: 1 dargestellten Sequenz im Rahmen der Degeneration des genetischen Codes entspricht, wobei die Veränderungen vorzugsweise eine im jeweiligen, zur Klonierung verwendeten Wirtsorganismus, insbesondere E.coli, verbesserte Codonnutzung bringen soll. Die für die einzelnen Wirtsorganismen geeignete Codonnutzung ist dem Fachmann bekannt.

Weiterhin beinhaltet die Erfindung eine DNA-Sequenz, die unter stringenten Hybridisierungsbedingungen mit der in SEQ ID NO: 1 dargestellten Sequenz oder/und einer davon im Rahmen der Degeneration des genetischen Codes abgeleiteten Sequenz hybridisiert. Unter stringenten Hybridisierungsbedingungen hybridisierend im Sinne der vorliegenden Erfindung versteht man, wenn ein Hybridisierungssignal auch nach Waschen bei einer hohen Temperatur, insbesondere 65°C oder/und in einem Puffer mit gerin- gem Salzgehalt auftritt (siehe auch Maniatis et al., "Molecular Cloning. A laboratory manual" (1982), Cold Spring Harbor Laboratory, New York).

Unter einem Protein mit einer N-Carbamoylsarcosin-Amidohydrolase-Aktivität im Sinne der vorliegenden Erfindung versteht man neben der in SEQ ID NO: 2 dargestellten Aminosäuresequenz auch deren Variationen und Derivate, die durch Deletion, Insertion oder/und Austausch einzelner Aminosäuren, sowie durch Verbindung mit einer oder mehreren anderen Polypeptiddomänen (resultierend in einem Fusionsprotein) erhalten werden.

Die erfindungsgemäße rekombinante DNA ist durch Klonierung von Arthrobacter DNA-Fragmenten in einen Wirtsorganismus, vorzugsweise E.coli, und Selektion der erhaltenen Klone auf direkte Genexpression erhältlich. Die Identifizierung von CSHase kodierenden Klonen kann günstigerweise über einen Plattenaktivitätstest erfolgen. Dazu wird dem Medium Sarcosinoxidase, Peroxidase, N-Carbamoyl-Sarcosin, 4-Aminoantipyrin und Tribrom-3-hydroxybenzoesäure in Phosphat-gepufferter Agarose zugegeben. Das Meßprinzip ist wie folgt: Die von einem positiven Klon exprimierte CSHase wandelt zugesetztes N-Carbamoyl-Sarcosin in Sarcosin um, dieses wird durch Sarcosinoxidase abgebaut zu Glycin, Formaldehyd und Wasserstoffperoxid. Die Peroxidase wandelt mit Hilfe des entstandenen Wasserstoffperoxids die zugesetzten Farbsubstrate durch oxidative Kupplung in einen dunkelvioletten Farbstoff um. Auf diese Weise erkennt man positive Klone durch Verfärbung.

Ein Gegenstand der vorliegenden Erfindung ist somit auch ein rekombinanter Vektor, der als heterologe Insertion mindestens eine Kopie einer rekombinanten DNA nach Anspruch 1 enthält. Der erfindungsgemäße Vektor kann ein eukaryontischer oder prokaryontischer oder ein sowohl in Eukaryonten als auch in Prokaryonten exprimierbarer Vektor sein. Er ist vorzugsweise ein prokaryontischer Vektor.

Weiterhin kann der erfindungsgemäße Vektor ein Vektor sein, der in einer Wirtszelle extrachromosomal vorliegt oder sich in das Chromosom der Wirtszelle integriert. Ein Beispiel für einen sich in das Genom der Wirtszelle integrierenden Vektor ist z.B. ein Bacteriophage λ Vektor in einer E.coli-Zelle. Ein Beispiel für einen extrachromosomal vorliegenden Vektor ist ein Plasmid. Vorzugsweise ist der erfindungsgemäße Vektor ein Plasmid.

Die Expression von CSHase durch ein 5 kb langes in E.coli kloniertes Arthrobacter-DNA-Fragment erwies sich als äußerst instabil. Bereits nach 24 Stunden konnte keine Expression von CSHase in den ursprünglich positiven Klonen nachgewiesen werden. Überraschenderweise zeigte sich jedoch, daß nach einer Verkürzung des Arthrobacter-DNA-Fragments auf eine Länge von 1,3 kB die Instabilität beseitigt werden konnte. Vorzugsweise betrifft die vorliegende Erfindung einen rekombinanten E.coli Expressionsvektor, der mindestens eine Kopie einer heterologen Insertion enthält, wobei die heterologe Insertion eine für ein Protein mit einer N-Cabamoylsarcosin-Amidohydrolase-Aktivität kodierende DNA-Sequenz enthält, eine Länge von nicht mehr als 1,3 kb aufweist und
(1) die in SEQ ID NO. 1 dargestellte Sequenz,
(2) eine dieser Sequenz im Rahmen der Degeneration des genetischen Codes entsprechende Sequenzen oder
(3) eine mit einer Sequenz aus (1) oder/und (2) unter stringenten Hybridisierungsbedingungen hybridisierende Sequenz enthält.

Auf einem erfindungsgemäßen Vektor befindet sich die heterologe Insertion günstigerweise unter Kontrolle eines Expressionssignals, um die CSHase-Expression in einem mit dem erfindungsgemäßen Vektor transformierten Wirtsorganismus zu ermöglichen. Dieses Expressionssignal muß für den jeweils verwendeten Wirtsorganismus geeignet sein. Für die besonders bevorzugte Expression in E.coli ist hierfür einerseits ein induzierbares Expressionssignal geeignet. Beispielsweise kann man die bekannten induzierbaren E.coli-Promotoren lac, tac oder trp, oder vorzugsweise den Salmonella-Promotor mgl (WO 88/09373) verwenden, bei dem es sich um ein Expressionssignal handelt, das bezüglich des E.coli-Vektors heterolog ist.

Andererseits hat sich überraschenderweise auch ein Expressionssignal als gut geeignet erwiesen, das zur Insertion homolog ist, d.h. ein Expressionssignal, das aus Arthrobacter stammt. Besonders bevorzugt wird hierfür der authentische Promotor des N-Carbamoylsarcosin-Amidohydrolase-Gens oder eine davon abgeleitete Sequenz verwendet, der überraschenderweise in E.coli eine sehr hohe Aktivität zeigt.

Diese Promotorregion ist auf einem 373 Basenpaare langen DNA-Fragment enthalten, das in SEQ ID NO: 3 dargestellt ist. Es wurde festgestellt, daß nicht nur dieses 373 bp lange Fragment, sondern auch verkürzte oder davon abgeleitete Fragmente Promotoreigenschaften besitzen.

Weiterhin ein Gegenstand der vorliegenden Erfindung ist eine Zelle, die mit einer erfindungsgemäßen DNA oder einem erfindungsgemäßen Vektor transformiert ist. Vorzugsweise ist diese Zelle eine Bakterienzelle, besonders bevorzugt eine E.coli- Zelle.

Weiterhin ein Gegenstand der vorliegenden Erfindung ist ein Verfahren zur Gewinnung eines Proteins mit N-Carbamoylsarcosin-Amidohydrolase-Aktivität, worin man
(1) eine Wirtszelle mit einer erfindungsgemäßen DNA oder einem Vektor transformiert, wobei eine transformierte Wirtszelle entsteht, die mindestens eine Kopie des N-Carbamoylsarcosin-Amidohydrolase-Gens enthält,
(2) transformierte Wirtszellen in einem geeigneten Medium kultiviert,
(3) das N-Carbamoylsarcosin-Amidohydrolase-Gen in den transformierten Wirtszellen zur Expression bringt und
(4) das Expressionsprodukt aus dem Medium oder den Wirtszellen gewinnt.

Die Transformation der Wirtszelle und die Kultivierung der transformierten Wirtszellen kann dabei nach an sich dem Fachmann auf dem Gebiet der Molekularbiologie bekannten Verfahren erfolgen. Die Expression des N-Carbamoylsarcosin-Amidohydrolase-Gens in den transformierten Wirtszellen erfolgt durch einen Induktionsschritt, sofern man ein induzierbares Expressionssignal verwendet, oder konstitutiv. Die Anreicherung des Expressionsprodukts aus dem Medium oder den Wirtszellen erfolgt vorzugsweise nach Zellaufschluß über DEAE-Sephadex und Phenyl-Sepharose-Chromatographie mit anschließender Ammoniumsulfatfällung, wie z.B. in EP-A 0 112 571 unter Beispiel 2b beschrieben.

Vorzugsweise verwendet man bei dem erfindungsgemäßen Verfahren als Wirtszellen E.coli-Bakterien. Weiterhin bevorzugt ist die Verwendung eines Vektors, der mindestens eine Kopie einer heterologen Insertion enthält, wobei die heterologe Insertion eine für ein Protein mit einer N-Cabamoylsarcosin-Amidohydrolase-Aktivität kodierende DNA-Sequenz enthält, eine Länge von nicht mehr als 1,3 kb aufweist und
(1) die in SEQ ID NO. 1 dargestellte Sequenz,
(2) eine dieser Sequenz im Rahmen der Degeneration des genetischen Codes entsprechende Sequenzen oder
(3) eine mit einer Sequenz aus (1) oder/und (2) unter stringenten Hybridisierungsbedingungen hybridisierende Sequenz enthält.

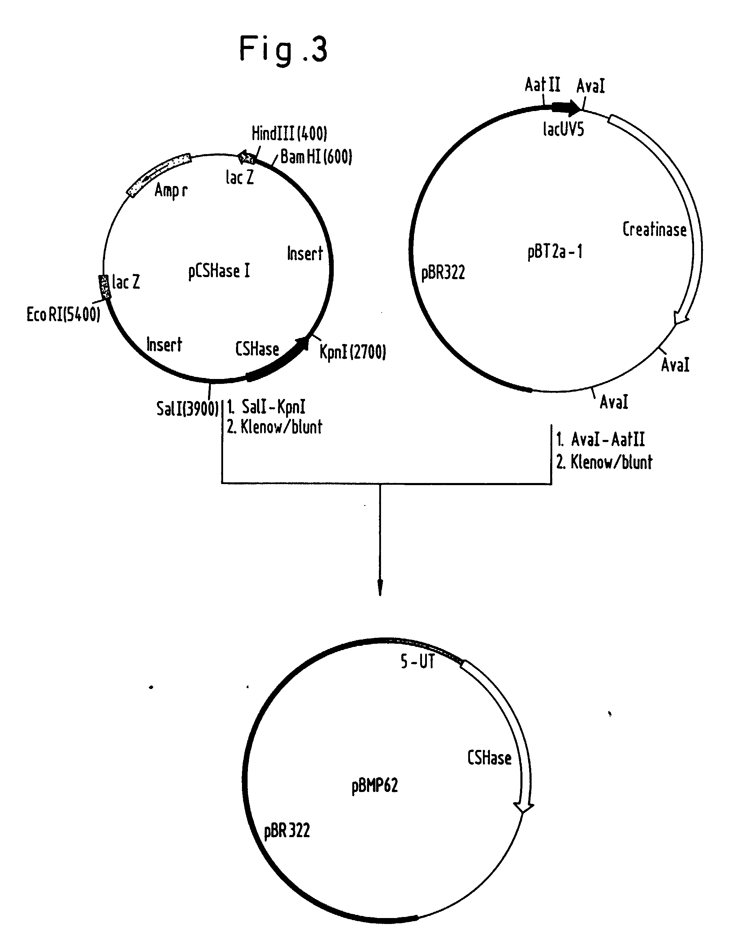

Besonders bevorzugt ist die Verwendung eines Vektors, bei dem die Expression des N-Carbamoylsarcosin-Amidohydrolase-Gens unter Kontrolle des authentischen Promotors oder einer davon abgeleiteten Sequenz erfolgt. Ein Beispiel für einen solchen Vektor ist pBMP62 (Figur 3).

Weiterhin ein Gegenstand der vorliegenden Erfindung ist das Protein mit N-Carbamoylsarcosin-Amidohydrolase-Aktivität, welches durch das erfindungsgemäße Verfahren erhalten wird. Vorzugsweise besitzt ein solches Protein die in SEQ ID NO: 2 dargestellte Aminosäuresequenz. Es können jedoch auch Derivate und Variationen dieses Proteins durch das erfindungsgemäße Verfahren erhalten werden.

Weiterhin beinhaltet die vorliegende Erfindung die Verwendung des erfindungsgemäßen Proteins zur Bestimmung von Creatinin, sowie ein Reagenz zur Creatininbestimmung, welches das erfindungsgemäße Protein mit N-Carbamoylsarcosin-Amidohydrolase-Aktivität enthält.

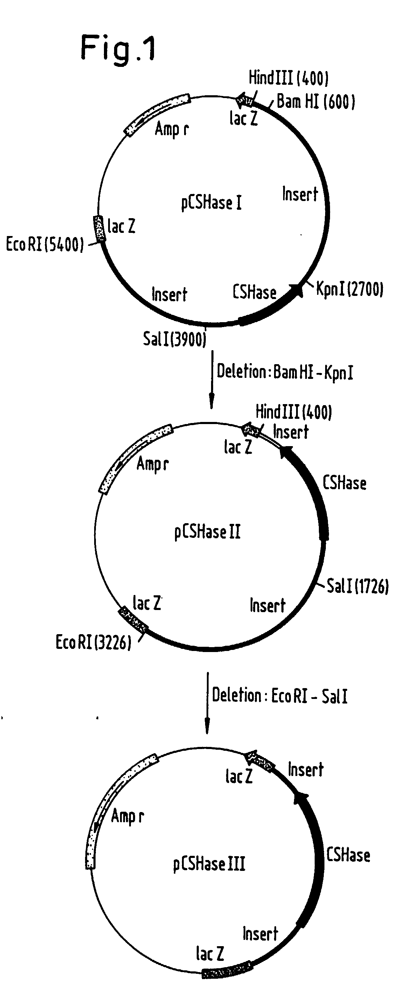

Die Erfindung wird durch die nachfolgenden Beispiele in Verbindung mit den Sequenzprotokollen und Figuren 1 bis 3 weiter verdeutlicht. Es zeigen
- SEQ ID NO: 1: die DNA-Sequenz des CSHase-Gens,
- SEQ ID NO: 2: die Aminosäuresequenz der CSHase,
- SEQ ID NO: 3: das als Promotor für das CSHase-Gen wirksame DNA-Fragment,

- Figur 1: die Konstruktion des stabilen Plasmids pCSHaseIII aus pCSHaseI durch Deletion,
- Figur 2: die Konstruktion des Expressionsvektors pBMP61,
- Figur 3: die Konstruktion des Expressionsvektors pBMP62.

Die in Beschreibung und Beispielen erwähnten Mikroorganismen und Plasmide

| | |
|---|---|
| Arthrobacter spec. | DSM 2563 |
| E.coli ED 8654 | DSM 2102 |
| pBT 2a-1 | DSM 3148p |

sind bei Deutsche Sammlung von Mikroorganismen und Zellkulturen GmbH (DSM), Mascheroder Weg 1B, D-3300 Braunschweig hinterlegt.

### Beispiel 1

### Identifizierung positiver Klone

Aus Arthrobacter spec. DSM 2563 wurde DNA nach gängigen Methoden isoliert (J.Marmur, J.Mol.Biol. 3 (1961) 208-218; S.Visuvanathan et al., J.Microbiol.Meth. 10 (1989) 59-64). Die auf diese Weise isolierte DNA wurde vollständig mit den Restriktionsenzymen EcoRI und HindIII gespalten. Der kommerziell erhältliche Vektor pUC18 (Boehringer Mannheim GmbH) wurde ebenfalls mit EcoRI/HindIIIgespalten und mit den DNA-Fragmenten aus Arthrobacter ligiert. Mit den resultierenden rekombinanten Plasmiden wurden kompetente E.coli ED 8654-Zellen (DSM 2102) transformiert (Vorschrift: Sambrook, Fritsch, Maniatis - Molecular Cloning. Second Edition 1.74 ff.). Nach Selektion auf Ampicillin wurden ca. 200 Kolonien pro Selektionsplatte erhalten. Die Identifizierung von CSHase-kodierenden Klonen erfolgte mittels eines in EP-A 0 154 269 beschriebenen Farbtests, bei dem Sarcosinoxidase, Peroxidase, Carbamoylsarcosin, 4-Aminoantipyrin und Tribrom-3-hydroxybenzoesäure in Phosphatpuffer pH 7,8 zugesetzt wurden.

Bei diesem Farbtest wandelt CSHase das zugesetzte Carbamoylsarcosin in Sarcosin um, dieses wird durch die Sarcosinoxidase zu Glycin, Formaldehyd und Wasserstoffperoxid abgebaut.

Die Peroxidase wandelt mit Hilfe des entstandenen Wasserstoffperoxids die zugesetzten Farbsubstrate in einen dunkelvioletten Farbstoff um. Die Extinktionszunahme wird bei 546 nm gemessen. Pro 1000 Kolonien wurden zwei positive Klone im Plattenaktivitätstest identifiziert.

### Beispiel 2

### Isolierung von CSHase kodierender DNA

Die DNA aus den positiven Klonen wurde nach Ausstrich der Kolonien auf Agar-Platten und Resuspendierung der Klone von diesen Agarplatten isoliert. Retransformation zeigte, daß zwar kurzfristig eine Expression in E.coli erfolgt, daß jedoch diese Plasmide eine äußerst hohe Instabilität aufweisen. Die im Plattenaktivitätstest nachweisbare Enzymaktivität ging rasch verloren. Es gelang jedoch, aus diesem Plattenlysat ein 5 kb große EcoRI/HindIII-DNA-Fragment zu isolieren, welches das CSHase-Gen enthält. Eine Verkürzung dieses DNA-Fragments auf 2,5 kb durch Schnitt mit den Restriktionsenzymen KpnI und BamHI und weiter mit EcoRI/SalI auf 1,3 kb führte zu stabilen Klonen.

Figur 1 zeigt das durch Klonierung des 5 kb Arthrobacter-DNA-Fragments in pUC18 erhaltene rekombinante Plasmid pCSHaseI und das daraus durch Deletion resultierende stabile Plasmid pCSHaseIII.

### Beispiel 3

### Konstruktion des Expressionsvektors pBMP61

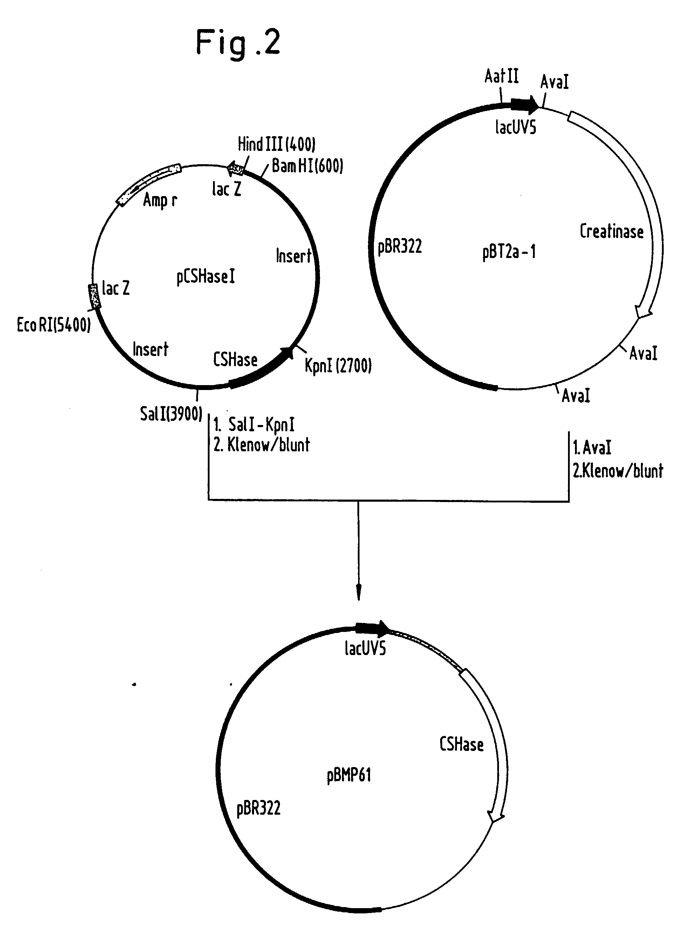

Die für CSHase kodierende DNA wurde in den Vektor pBT2a-1 (DSM 3148P) kloniert. Dafür wurde zunächst aus pBT2a-1 das Creatinase-Gen durch Spaltung mit AvaI deletiert. Die Enden des resultierenden DNA-Fragments wurden mit Klenow-Enzym und den vier Nukleotidtriphosphaten glatt gemacht. Aus pCSHaseI wurde das CSHase-Gen mit den Restriktionsenzymen SalI und KpnI herausgeschnitten, mit Klenow-Enzym und den vier Nukleotidtriphosphaten glatt gemacht und mit dem 2,6 kb großen AvaI-Fragment von pBT2a-1 zu dem resultierenden rekombinanten Plasmid pBMP61 ligiert (siehe Figur 2).

### Beispiel 4

### Konstruktion des Expressionsvektors pBMP62

Das Plasmid pBMP61 enthält noch den lacUV5 Promotor aus dem Plasmid pBT2a-1. Dieser Promotor ist für die Expression nicht notwendig. Daher wurde pBT2a-1 mit AatII und AvaI gespalten und das resultierende 2,4 kb große AvaI/AatII-Fragment mit Klenow-Enzym und den vier Nukleotidtriphosphaten glatt gemacht. Das resultierende 2,4 kb große glattendige Fragment wurde dann mit dem ebenfalls glattgemachten 1,2 kb großen SalI/KpnI-Fragment aus pCSHaseI zu Plasmid pBMP62 ligiert (Figur 3). Dieses für CSHase kodierende Plasmid enthält noch ca. 373 bp einer Arthrobacter-Sequenz in der 5'-nichttranslatierten Region des CSHase-Gens, die in SEQ ID NO: 3 dargestellt ist.

### Beispiel 5

### Expression der CSHase in E.coli

Die CSHase-kodierenden Plasmide pBMP61 oder pBMP62 wurden in E.coli ED 8654 (DSM 2102) transformiert (Vorschrift: Sambrook, Fritsch, Maniatis - Molecular Cloning. Second Edition 1.74 ff.) und auf Ampicillin selektioniert. Nach Anzucht der Klone in 5 ml LB über Nacht wurden die Zellen geerntet und mit Ultraschall aufgeschlossen und anschließend ein Aktivitätstest durchgeführt. Das Meßprinzip ist dasselbe wie in dem Plattenaktivitätstest von Beispiel 1. Es wird die Extinktionszunahme bei einer Wellenlänge von 546 nm gemessen. Der Aktivitätstest ist in der EP-A 0 154 269 beschrieben. Eine Einheit (U) ist definiert als µmol Sarcosinbildung pro min bei 25°C unter den Meßbedingungen im gekoppelten Test mit Sarcosinoxidase und Peroxidase.

Mit dem rekombinanten Enzym wird eine Aktivität von 150 U/l x OD erhalten. Dies entspricht einer Steigerung gegenüber der Ausgangskultur (Arthrobactersp. DSM 2563) um einen Faktor von ca. 50.

| Stamm | Aktivität [U/l x OD] |
|---|---|
| Arthrobacter sp.DSM 2563 | 3 |
| E.coli | 0 |
| E.coli/pBMP61 | 150 |
| E.coli/pBMP62 | 150 |

### Beispiel 6

### Sequenzierung des CSHase-Gens

Das 1,3 kb große Arthrobacter-DNA-Fragment aus pCSHaseIII, welches das CSHase-Gen enthält, wurde zur Erstellung der DNA-Sequenz verwendet. Die DNA wurde in M13 subkloniert und nach Standardtechniken (Didesoxymethode nach Sanger) sequenziert. Es ergibt sich ein offenes Leseraster, dessen DNA-Sequenz in SEQ ID NO: 1 dargestellt ist. Es kodiert für ein Protein mit 264 Aminosäuren, dessen Sequenz in SEQ ID NO: 2 dargestellt ist.
SEQ ID NO:1
   SEQUENZLÄNGE: 795 Basenpaare
   ART DER SEQUENZ: Nukleotidsequenz (CSHase-Sequenz) 
ATGACTGAAA CATCGGGAAC CTTCAACGAC ATTGAAGCTC GACTAGCCGC TGTGCTGGAA 60
GAAGCATTCG AAGCCGGCAC GAGCATCTAC AACGAGCGCG GTTTCAAGCG TCGAATCGGC 120
TACGGCAACC GTCCCGCCGT CATCCATATC GACCTCGCTA ACGCATGGAC TCAACCCGGG 180
CACCCCTTCA GCTGCCCGGG CATGGAGACG ATCATCCCGA ACGTGCAGCG GATCAACGAA 240
GCTGCACGCG CCAAAGGGGT CCCGGTCTTC TACACCACCA ACGTGTACCG GAATCGCGAT 300
GCCAGCTCCG GGACCAACGA TATGGGCCTG TGGTACTCGA AGATCCCCAC CGAAACCCTG 360
CCGGCAGACT CCTACTGGGC GCAGATCGAT GACCGCATCG CCCCCGCAGA TGGTGAAGTC 420
GTGATCGAGA AAAACCGTGC CTCGGCATTC CCGGGAACGA ATTTGGAGCT CTTCCTGACG 480
TCCAACCGCA TCGACACCCT CATCGTGACC GGGGCAACCG CGGCAGGCTG CGTGCGCCAC 540
ACTGTCGAGT ACGCAATCGC CAAGGGATTC CGCCCGATCA TTCCACGCGA AACCATCGGC 600
GACCGCGTGC CAGGTGTTGT GCAGTGGAAC CTTTACGACA TCGACAACAA GTTTGGTGAC 660
GTGGAGTCTA CCGATTCGGT GGTGCAATAC TTGTACGCAC TTCCGCAGTT CGAAGACACC 720
GTTCCGAAGA CCCTATCCGA TCCCCAGCCT GAGGTAGAGG CTCCGGCAGA CCCGGTCTTC 780
GCTGAGCAGC ACTAA 795
SEQ ID NO:2
   SEQUENZLÄNGE: 264 Aminosäuren
   ART DER SEQUENZ: Aminosäuresequenz (CSHase-Proteinsequenz) 
Met Thr Glu Thr Ser Gly Thr Phe Asn Asp Ile Glu Ala Arg Leu Ala
                5                   10                  15

Ala Val Leu Glu Glu Ala Phe Glu Ala Gly Thr Ser Ile Tyr Asn Glu
            20                  25                  30

Arg Gly Phe Lys Arg Arg Ile Gly Tyr Gly Asn Arg Pro Ala Val Ile
        35                  40                  45

His Ile Asp Leu Ala Asn Ala Trp Thr Gln Pro Gly His Pro Phe Ser
    50                  55                  60

Cys Pro Gly Met Glu Thr Ile Ile Pro Asn Val Gln Arg Ile Asn Glu
65                  70                  75                  80

Ala Ala Arg Ala Lys Gly Val Pro Val Phe Tyr Thr Thr Asn Val Tyr
                85                  90                  95

Arg Asn Arg Asp Ala Ser Ser Gly Thr Asn Asp Met Gly Leu Trp Tyr
            100                 105                 110

Ser Lys Ile Pro Thr Glu Thr Leu Pro Ala Asp Ser Tyr Trp Ala Gln
        115                 120                 125

Ile Asp Asp Arg Ile Ala Pro Ala Asp Gly Glu Val Val Ile Glu Lys
    130                 135                 140

Asn Arg Ala Ser Ala Phe Pro Gly Thr Asn Leu Glu Leu Phe Leu Thr
145                 150                 155                 160

Ser Asn Arg Ile Asp Thr Leu Ile Val Thr Gly Ala Thr Ala Ala Gly
                165                 170                 175

Cys Val Arg His Thr Val Glu Tyr Ala Ile Ala Lys Gly Phe Arg Pro
            180                 185                 190

Ile Ile Pro Arg Glu Thr Ile Gly Asp Arg Val Pro Gly Val Val Gln
        195                 200                 205

Trp Asn Leu Tyr Asp Ile Asp Asn Lys Phe Gly Asp Val Glu Ser Thr
    210                 215                 220

Asp Ser Val Val Gln Tyr Leu Tyr Ala Leu Pro Gln Phe Glu Asp Thr
225                 230                 235                 240

Val Pro Lys Thr Leu Ser Asp Pro Gln Pro Glu Val Glu Ala Pro Ala
                245                 250                 255

Asp Pro Val Phe Ala Glu Gln His
            260
SEQ ID NO:3
   SEQUENZLÄNGE: 373 Basenpaare
   ART DER SEQUENZ: Nukleotidsequenz (CSHase:5'untranslatierte Region)(-373 bis -1)

GTCGACATCC AGCAGATCTG GCATCTGATA CGAACAGCGC CGCTTGAGCA CTCGGTACTA 60
CTTTTTGAAC CGCTAGATAT CTTAGTTTTC AAATACTGCC AGAGGGAACT CCCTACCCCA 120
TGGCAACCAT CTTTACTGCT GGTCATGACG CGCATGGATT CGCTCAAATG GGTCACAGCT 180
TGCTGCCATC CGAGAGCTAG AAGCCGTAAT TTCCCAGCGG TTTTTGAATT ATTCGCATCT 240
CGACAAGGCC TTTTACTAAC AAAAAATATC TTTTCCCGAA CCACTAGTGC CTCAGCAATC 300
CTGGGCTATA GYACTTACCA GAGCAACGAG GTTGCTTTAT CAGCAACAAA CCAAGATGAG 360
AATAGAGAGA GCA 373

## Patentansprüche (Patentansprüche für folgende(n) Vertragsstaat(en): AT, BE, CH, LI, DE, DK, FR, GB, GR, IT, LU, NL, SE)

1. Rekombinante DNA, **dadurch gekennzeichnet,**
daß sie die in SEQ ID NO. 1 dargestellte Sequenz enthält, wobei die DNA-Sequenz für ein Protein mit einer N-Carbamoylsarcosin-Amidohydrolase-Aktivität kodiert.

2. Rekombinanter Vekcor,
**dadurch gekennzeichnet,**
daß er als heterologe Insertion mindestens eine Kopie einer rekombinanten DNA nach Anspruch 1 enthält.

3. Rekombinanter E.coli Expressionsvektor, der mindestens eine Kopie einer heterologen Insertion enthält,
**dadurch gekennzeichnet,**
daß die heterologe Insertion eine für ein Protein mit einer N-Carbamoylsarcosin-Amidohydrolase-Aktivität kodierende DNA-Sequenz enthält, eine Länge von nicht mehr als 1,3 kb aufweist und
(1) die in SEQ ID NO. 1 dargestellte Sequenz,
(2) eine dieser Sequenz im Rahmen der Degeneration des genetischen Codes entsprechende Sequenzen oder
(3) eine mit einer Sequenz aus (1) oder/und (2) unter stringenten Hybridisierungsbedingungen hybrisisierende Sequenz enthält.

4. Rekombinanter Vektor nach einem der Ansprüche 2 bis 3,
**dadurch gekennzeichnet,**
daß er als Expressionssignal den authentischen Promotor des N-Carbamoylsarcosin-Amidohydrolase-Gens oder eine davon abgeleitete Sequenz enthält.

5. Zelle,
**dadurch gekennzeichnet,**
daß sie mit einem Vektor nach einem der Ansprüche 2 bis 4 transformiert ist.

6. Verfahren zur Gewinnung eines Proteins mit N-Carbamoylsarcosin-Amidohydrolase-Aktivität,
**dadurch gekennzeichnet,** daß man
(1) eine Wirtszelle mit einer DNA nach Anspruch 1 oder einem Vektor nach einem der Ansprüche 2 bis 4 unter Bildung einer transformierten Wirtszelle transformiert, die mindestens eine Kopie des N-Carbamoylsarcosin-Amidohydrolase-Gens enthält,
(2) transformierte Wirtszellen in einem geeigneten Medium kultiviert,
(3) das N-Carbamoylsarcosin-Amidohydrolase-Gen in den transformierten Wirtszellen zur Expression bringt und
(4) das Expressionsprodukt aus dem Medium oder den Wirtszellen gewinnt.

7. Verfahren nach Anspruch 6,
**dadurch gekennzeichnet,**
daß man einen Vektor verwendet, bei dem die Länge der das N-Carbamoylsarcosin-Amidohydrolase-Gen enthaltenden heterologen Insertion nicht mehr als 1,3 kb beträgt.

8. Verfahren nach einem der Ansprüche 6 bis 7,
**dadurch gekennzeichnet,**
daß man einen Vektor verwendet, bei dem die Expression des N-Carbamoylsarcosin-Amidohydrolase-Gens unter Kontrolle des authentischen Promotors oder einer davon abgeleiteten Sequenz erfolgt.

## Patentansprüche (Patentansprüche für folgende(n) Vertragsstaat(en): ES)

1. Verfahren zur Herstellung einer rekombinanten DNA, die die in SEQ ID NO. 1 dargestellte Sequenz enthält, wobei die DNA-Sequenz für ein Protein mit einer N-Carbamoylsarcosin-Amidohydrolase-Aktivität kodiert,
**dadurch gekennzeichnet,**
daß man Arthrobacter DNA-Fragmente in einen Wirtsorganismus kloniert und die erhaltenen Klone auf Genexpression selektioniert.

2. Verfahren zur Herstellung eines rekombinanten Vektors,
**dadurch gekennzeichnet,**
daß man in einen Ausgangsvektor als heterologe Insertion mindestens eine Kopie einer rekombinanten DNA einführt, die die in SEQ ID NO. 1 dargestellte Sequenz enthält, wobei die DNA-Sequenz für ein Protein mit einer N-Carbamoylsarcosin-Amidohydrolase-Aktivität kodiert.

3. Verfahren zur Herstellung eines rekombinanten E.coli Expressionsvektors, der mindestens eine Kopie einer heterologen Insertion enthält, **dadurch gekennzeichnet,**
daß man in einen Ausgangsvektor als heterologe Insertion eine für ein Protein mit einer N-Carbamoylsarcosin-Amidohydrolase-Aktivität kodierende DNA-Sequenz einführt, die eine Länge von nicht mehr als 1,3 kb aufweist und
(1) die in SEQ ID NO. 1 dargestellte Sequenz,
(2) eine dieser Sequenz im Rahmen der Degeneration des genetischen Codes entsprechende Sequenzen oder
(3) eine mit einer Sequenz aus (1) oder/und (2) unter stringenten Hybridisierungsbedingungen hybridisierende Sequenz enthält.

4. Verfahren nach einem der Ansprüche 2 bis 3,
**dadurch gekennzeichnet,**
daß der Vektor als Expressionssignal den authentischen Promotor des N-Carbamoylsarcosin-Amidohydrolase-Gens oder eine davon abgeleitete Sequenz enthält.

5. Verfahren zur Herstellung einer Zelle,
**dadurch gekennzeichnet,**
daß man eine Wirtszelle mit einem Vektor erhalten nach einem der Ansprüche 2 bis 4 transformiert.

6. Verfahren zur Gewinnung eines Proteins mit N-Carbamoylsarcosin-Amidohydrolase-Aktivität,
**dadurch gekennzeichnet,**
daß man
(1) eine Wirtszelle mit einer DNA, die die in SEQ IN NO. 1 dargestellte Sequenz enthält, einem mindestens eine Kopie dieser DNA als heterologe Insertion enthaltenden rekombinanten Vektor oder einem rekombinanten E.coli Expressionsvektor, der mindestens eine Kopie einer heterologen Insertion mit einer für ein Protein mit einer N-Carbamoylsarcosin-Amidohydrolase-Aktivität kodierenden DNA-Sequenz, die eine Länge von nicht mehr als 1,3 kb aufweist und
(1) die in SEQ ID NO. 1 dargestellte Sequenz,
(2) eine dieser Sequenz im Rahmen der Degeneration des genetischen Codes entsprechende Sequenzen oder
(3) eine mit einer Sequenz aus (1) oder/und (2) unter stringenten Hybridisierungsbedingungen hybridisierende Sequenz enthält,
unter Bildung einer transformierten Wirtszelle transformiert, die mindestens eine Kopie des N-Carbamoylsarcosin-Amidohydrolase-Gens enthält,
(2) transformierte Wirtszellen in einem geeigneten Medium kultiviert,
(3) das N-Carbamoylsarcosin-Amidohydrolase-Gen in den transformierten Wirtszellen zur Expression bringt und
(4) das Expressionsprodukt aus dem Medium oder den Wirtszellen gewinnt.

7. Verfahren nach Anspruch 6,
**dadurch gekennzeichnet,**
daß man einen Vektor verwendet, bei dem die Länge der das N-Carbamoylsarcosin-Amidohydrolase-Gen enthaltenden heterologen Insertion nicht mehr als 1,3 kb beträgt.

8. Verfahren nach einem der Ansprüche 6 bis 7,
**dadurch gekennzeichnet,**
daß man einen Vektor verwendet, bei dem die Expression des N-Carbamoylsarcosin-Amidohydrolase-Gens unter Kontrolle des authentischen Promotors oder einer davon abgeleiteten Sequenz erfolgt.

## Claims (Claims for the following Contracting State(s): AT, BE, CH, LI, DE, DK, FR, GB, GR, IT, LU, NL, SE)

1. Recombinant DNA,
**wherein**
it contains the sequence shown in SEQ ID NO.1 wherein the DNA sequence codes for a protein with an N-carbomoyl sarcosine amidohydrolase activity.

2. Recombinant vector,
**wherein**
it contains at least one copy of a recombinant DNA as claimed in claim 1 as a heterologous insertion.

3. Recombinant E. coli expression vector which contains at least one copy of a heterologous insertion,
**wherein**
the heterologous inserion contains a DNA sequence coding for a protein with an N-carbamoyl sarcosine amidohydrolase activity, has a length of not more than 1.3 kb and contains
(1) the sequence shown in SEQ ID NO 1
(2) a sequence corresponding to it within the scope of the degeneracy of the genetic code or
(3) a sequence which hybridizes with a sequence from (1) or/and (2) under stringent hybridizing conditions.

4. Recombinant vector as claimed in one of the claims 2 to 3,
**wherein**
it contains the authentic promoter of the N-carbamoyl sarcosine amidohydrolase gene or a sequence derived therefrom as the expression signal.

5. Cell,
**wherein**
it is transformed with a vector as claimed in one of the claims 2 to 4.

6. Process for the isolation of a protein with N-carbamoyl sarcosine amidohydrolase activity,
**wherein**
(1) a cell is transformed with a DNA as claimed in claim 1 or with a vector as claimed in one the claims 2 to 4 whereby a transformed host cell is formed which contains at least one copy of the N-carbamoyl sarcosine amidohydrolase gene,
(2) the transformed host cells are cultured in a suitable medium,
(3) the N-carbamoyl sarcosine amidohydrolase gene is expressed in the transformed host cells and
(4) the expression product is isolated from the medium or the host cells.

7. Process as claimed in claim 6,
**wherein**
a vector is used in which the length of the heterologous insertion containing the N-carbamoyl sarcosine amidohydrolase gene is not more than 1.3 kb.

8. Process as claimed in one of the claims 6 to 7,
**wherein**
a vector is used in which the expression of the N-carbamoyl sarcosine amidohydrolase gene is under the control of the authentic promoter or of a sequence derived therefrom.

## Claims (Claims for the following Contracting State(s): ES)

1. Process for the production of a recombinant DNA containing the sequence shown in SEQ ID NO.1, in which the DNA sequence codes for a protein with an N-carbamoylsarcosine amidohydrolase activity
**wherein**
arthrobacter DNA fragments are cloned into a host organism and the clones obtained are selected for gene expression.

2. Process for the production of a recombinant vector,
**wherein**
at least one copy of a recombinant DNA containing the sequence shown in SEQ ID NO.1, in which the DNA sequence codes for a protein with an N-carbamoylsarcosine amidohydrolase activity, is introduced into a starting vector as a heterologous insertion.

3. Process for the production of a recombinant E. coli expression vector which contains at least one copy of a heterologous insertion,
**wherein**
a DNA sequence coding for a protein with an N-carbamoylsarcosine amidohydrolase activity is introduced into a starting vector as a heterologous insertion, in which the said DNA sequence has a length of not more than 1.3 kb and contains
(1) the sequence shown in SEQ ID NO 1
(2) a sequence corresponding to this sequence within the scope of the degeneracy of the genetic code or
(3) a sequence which hybridizes with a sequence from (1) or/and (2) under stringent hybridizing conditions.

4. Process as claimed in one of the claims 2 to 3,
**wherein**
the vector contains the authentic promoter of the N-carbamoylsarcosine amidohydrolase gene or a sequence derived therefrom as the expression signal.

5. Process for the production of a cell,
**wherein**
a host cell is transformed with a vector which is obtained as claimed in one of the claims 2 to 4.

6. Process for the isolation of a protein with N-carbamoylsarcosine amidohydrolase activity,
**wherein**
(1) a host cell is transformed with a DNA which contains the sequence shown in SEQ ID NO.1, a recombinant vector which contains at least one copy of this DNA as a heterologous insertion or a recombinant E. coli expression vector which has at least one copy of a heterologous insertion containing a DNA sequence coding for a protein with an N-carbamoylsarcosine amidohydrolase activity which has a length of not more than 1.3 kb and contains
(1) the sequence shown in SEQ ID NO. 1
(2) a sequence corresponding to this sequence within the scope of the degeneracy of the genetic code or
(3) a sequence which hybridizes with a sequence from (1) or/and (2) under stringent hybridizing conditions
to form a transformed host cell which contains at least one copy of the N-carbamoylsarcosine amidohydrolase gene
(2) the transformed host cells are cultured in a suitable medium,
(3) the N-carbamoylsarcosine amidohydrolase gene is expressed in the transformed host cells and
(4) the expression product is isolated from the medium or the host cells.

7. Process as claimed in claim 6,
**wherein**
a vector is used in which the length of the heterologous insertion containing the N-carbamoylsarcosine amidohydrolase gene is not more than 1.3 kb.

8. Process as claimed in one of the claims 6 to 7,
**wherein**
a vector is used in which the expression of the N-carbamoylsarcosine amidohydrolase gene is under the control of the authentic promoter or of a sequence derived therefrom.

## Revendications (Revendications pour l'(les) Etat(s) contractant(s) suivant(s): AT, BE, CH, LI, DE, DK, FR, GB, GR, IT, LU, NL, SE)

1. ADN recombiné caractérisé en ce qu'il contient la séquence représentée dans SEQ ID NO. 1, où la séquence d'ADN code une protéine ayant une activité de N-carbamoylsarcosine-amidohydrolase.

2. Vecteur recombiné caractérisé en ce qu'il contient comme insertion hétérologue au moins une copie d'un ADN recombiné selon la revendication 1.

3. Vecteur recombiné d'expression dans E. coli qui contient au moins une copie d'une insertion hétérologue caractérisé en ce que l'insertion hétérologue contient une séquence d'ADN codant une protéine ayant une activité de N-carbamoylsarcosine-amidohydrolase, présente une longueur d'au plus 1,3 kb et contient
(1) la séquence représentée dans SEQ ID NO. 1,
(2) une séquence correspondant à cette séquence dans le cadre de la dégénérescence du code génétique ou
(3) une séquence qui s'hybride avec une séquence de (1) et/ou (2) dans des conditions d'hybridation strictes.

4. Vecteur recombiné selon l'une des revendications 2 à 3 caractérisé en ce qu'il contient comme signal d'expression le promoteur authentique du gène de la N-carbamoylsarcosine-amidohydrolase ou une séquence dérivée de celui-ci.

5. Cellule caractérisée en ce qu'elle est transformée avec un vecteur selon l'une des revendications 2 à 4.

6. Procédé d'obtention d'une protéine à activité de N-carbamoylsarcosine-amidohydrolase caractérisé en ce que
(1) on transforme une cellule hôte avec un ADN selon la revendication 1 ou un vecteur selon l'une des revendications 2 à 4 avec formation d'une cellule hôte transformée qui contient au moins une copie du gène de la N-carbamoylsarcosine-amidohydrolase,
(2) on cultive les cellules hôtes transformées dans un milieu approprié,
(3) on amène à l'expression le gène de la N-carbamoylsarcosine-amidohydrolase dans les cellules hôtes transformées et
(4) on obtient le produit d'expression à partir du milieu ou des cellules hôtes.

7. Procédé selon la revendication 6 caractérisé en ce que l'on utilise un vecteur dans lequel la longueur de l'insertion hétérologue contenant le gène de la N-carbamoylsarcosine-amidohydrolase n'est pas supérieure à 1,3 kb.

8. Procédé selon l'une des revendications 6 à 7 caractérisé en ce que l'on utilise un vecteur dans lequel l'expression du gène de la N-carbamoylsarcosine-amidohydrolase a lieu sous le contrôle du promoteur authentique ou d'une séquence dérivée de celui-ci.

## Revendications (Revendications pour l'(les) Etat(s) contractant(s) suivant(s): ES)

1. Procédé de préparation d'un ADN recombiné qui contient la séquence représentée dans SEQ ID NO. 1, où la séquence d'ADN code une protéine ayant une activité de N-carbamoylsarcosine-amidohydrolase, caractérisé en ce que l'on clone des fragments d'ADN d'Arthrobacter dans un organisme hôte et on sélectionne les clones obtenus d'après l'expression génique.

2. Procédé de préparation d'un vecteur recombiné caractérisé en ce que l'on introduit dans un vecteur initial comme insertion hétérologue au moins une copie d'un ADN recombiné qui contient la séquence représentée dans SEQ ID NO. 1, où la séquence d'ADN code une protéine ayant une activité de N-carbamoylsarcosine-amidohydrolase.

3. Procédé de préparation d'un vecteur recombiné d'expression dans E. coli qui contient au moins une copie d'une insertion hétérologue caractérisé en ce que l'on introduit dans un vecteur initial comme insertion hétérologue une séquence d'ADN codant une protéine ayant une activité de N-carbamoylsarcosine-amidohydrolase qui présente une longueur d'au plus 1,3 kb et qui contient
(1) la séquence représentée dans SEQ ID NO. 1,
(2) une séquence correspondant à cette séquence dans le cadre de la dégénérescence du code génétique ou
(3) une séquence qui s'hybride avec une séquence de (1) et/ou (2) dans des conditions d'hybridation strictes.

4. Procédé selon l'une des revendications 2 à 3 caractérisé en ce que le vecteur contient comme signal d'expression le promoteur authentique du gène de la N-carbamoylsarcosine-amidohydrolase ou une séquence dérivée de celui-ci.

5. Procédé de préparation d'une cellule caractérisé en ce que l'on transforme une cellule hôte avec un vecteur obtenu selon l'une des revendications 2 à 4.

6. Procédé d'obtention d'une protéine à activité de N-carbamoylsarcosine-amidohydrolase caractérisé en ce que
(1) on transforme une cellule hôte avec un ADN qui contient la séquence représentée dans SEQ ID NO. 1, un vecteur recombiné contenant comme insertion hétérologue au moins une copie de cet ADN ou un vecteur recombiné d'expression dans E. coli qui contient au moins une copie d'une insertion hétérologue avec une séquence d'ADN codant une protéine ayant une activité de N-carbamoylsarcosine-amidohydrolase, qui présente une longueur d'au plus 1,3 kb et qui contient
(1) la séquence représentée dans SEQ ID NO. 1,
(2) une séquence correspondant à cette séquence dans le cadre de la dégénérescence du code génétique ou
(3) une séquence qui s'hybride avec une séquence de (1) et/ou (2) dans des conditions d'hybridation strictes,
avec formation d'une cellule hôte transformée qui contient au moins une copie du gène de la N-carbamoylsarcosine-amidohydrolase,
(2) on cultive les cellules hôtes transformées dans un milieu approprié,
(3) on amène à l'expression le gène de la N-carbamoylsarcosine-amidohydrolase dans les cellules hôtes transformées et
(4) on obtient le produit d'expression à partir du milieu ou des cellules hôtes.

7. Procédé selon la revendication 6 caractérisé en ce que l'on utilise un vecteur dans lequel la longueur de l'insertion hétérologue contenant le gène de la N-carbamoylsarcosine-amidohydrolase n'est pas supérieure à 1,3 kb.

8. Procédé selon l'une des revendications 6 à 7 caractérisé en ce que l'on utilise un vecteur dans lequel l'expression du gène de la N-carbamoylsarcosine-amidohydrolase a lieu sous le contrôle du promoteur authentique ou d'une séquence dérivée de celui-ci.
